(19) Europäisches Patentamt
European Patent Office
Office européen des brevets

(11) Veröffentlichungsnummer: **0 009 184**
**B1**

(12) # EUROPÄISCHE PATENTSCHRIFT

(45) Veröffentlichungstag der Patentschrift: **23.06.82**

(51) Int. Cl.³: **C 07 C 149/36**, C 08 G 63/68

(21) Anmeldenummer: **79103329.3**

(22) Anmeldetag: **07.09.79**

(54) **Bis-(4-hydroxyphenylthio)-benzol, seine Herstellung und Verwendung zur Herstellung von Polycarbonaten.**

(30) Priorität: **18.09.78 US 943516**
**18.09.78 US 943517**

(43) Veröffentlichungstag der Anmeldung: **02.04.80 Patentblatt 80/7**

(45) Bekanntmachung des Hinweises auf die Patenterteilung: **23.06.82 Patentblatt 82/25**

(84) Benannte Vertragsstaaten: **DE FR GB IT NL**

(56) Entgegenhaltungen:
**DE-OS-2 520 317**
**DE-OS-2 721 595**
**FR-A-2 134 505**
**GB-A-1 151 042**
**US-A-2 288 282**
**US-PS-3 250 744**
**US-A-3 269 986**
**US-PS-3 729 447**

(73) Patentinhaber: **Mobay Chemical Corporation, Penn Lincoln Parkway West, Pittsburgh, Pennsylvania 15205 (US)**

(72) Erfinder: **Baron, Arthur L., 211 East Thistle Court, New Martinsville, W.VA 26155 (US)**
Erfinder: **Sivaramakrishnan, Parameswar, 159 Fairview Drive, New Martinsville, W.VA 26155 (US)**

(74) Vertreter: **Gremm, Joachim, Dr. et al, Bayer AG c/o Zentralbereich Patente, Marken und Lizenzen, D-5090 Leverkusen 1, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Bis-(4-hydroxyphenylthio)-benzol, seine Herstellung und Verwendung zur Herstellung von Polycarbonaten

Gegenstand der vorliegenden Erfindung ist Bis-(4-hydroxyphenylthio)-benzol der Strukturformel

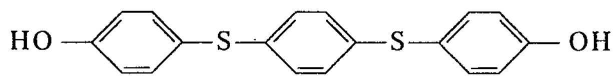

Gegenstand der vorliegenden Erfindung ist außerdem ein Verfahren zur Herstellung von Bis-(4-hydroxyphenylthio)-benzol, das dadurch gekennzeichnet ist, daß man 4-Mercaptophenol mit p-Dibrombenzol im Molverhältnis 2 : 1 in Gegenwart von Kaliumcarbonat und Dimethylformamid bei Temperaturen zwischen 90° C und 130° C umsetzt.

Gegenstand der vorliegenden Erfindung ist außerdem die Verwendung der Bis-(4-hydroxyphenylthio)-benzols zur Herstellung von Polycarbonaten.

Die Verwendung des erfindungsgemäßen Bis-(4-hydroxyphenylthio)-benzols zur Herstellung von aromatischen Polycarbonaten führt zu Produkten mit verbessertem Fließverhalten gegenüber vergleichbaren Produkten aus dem entsprechenden Bis-(4-hydroxyphenyl)-sulfid.

Gegenüber entsprechenden Copolycarbonaten aus Bisphenol-A und Bis-(4-hydroxyphenylsulfonyl)-benzol zeigen die vorstehenden Copolycarbonate aus dem erfindungsgemäßen Bisthioäther ebenfalls ein verbessertes Fließverhalten.

In der FR-PS 21 34 505 beziehungsweise in der entsprechenden US-PS 3 809 682 ist Bis-(4-hydroxyphenylthio)-benzol nicht explizit vorbeschrieben. Die vorliegende Erfindung wird auch aus diesen beiden Lit.-Stellen nicht nahegelegt, da gemäß diesen Lit.-Stellen die Herstellung von Polyäthern bzw. Polythioäthern gelehrt wird.

Aus der US-PS 3 269 986 sind Polycarbonate aus Dihydroxypolysulfonen bekannt. Wie oben erwähnt, haben jedoch Copolycarbonate aus den nächstvergleichbaren Bis-(4-hydroxyphenylsulfonyl)-benzol ein schlechteres Fließverhalten.

Bekannt sind auch Copolycarbonate aus Diphenolen, wie etwa Bisphenol-A, und Bis-(4-hydroxyphenyl)-sulfid (siehe US-PS 3 250 744 oder DE-OS 2 721 595); jedoch haben Copolycarbonate aus Bis-(4-hydroxyphenyl)-sulfid und Bisphenol-A ein schlechteres Fließverhalten.

Gegenüber diesem Stand der Technik sind GB-PS 1 151 042 und GB-PS 1 190 135 ferner liegend, da aliphatische Bindeglieder jeweils einbezogen sind.

Die Lehre der DE-OS 2 520 317 ist ebenfalls ferner liegend, da sie die Herstellung von halogenierten Polycarbonaten aus statischen halogenierten Bisphenol-Gemischen beschreibt, wobei unter vielen Varianten aus Bis-(4-hydroxyphenyl)-thioäther eingesetzt werden können.

Durch US-PS 3 729 447, Spalte 3, Formel VI wird der erfindungsgemäße Bis-thioäther auch nicht vorweggenommen, noch ist es aus der US-PS 3 729 447, die die Herstellung von Polyestercarbonaten beschreibt, naheliegend, daß die Verwendung des erfindungsgemäßen Bis-thioäthers zu Polycarbonaten mit verbessertem Fließverhalten führt.

Ein für die Synthese des Bis-(4-hydroxyphenylthio)-benzols geeignetes Verfahren wird durch das folgende allgemeine Reaktionsschema veranschaulicht:

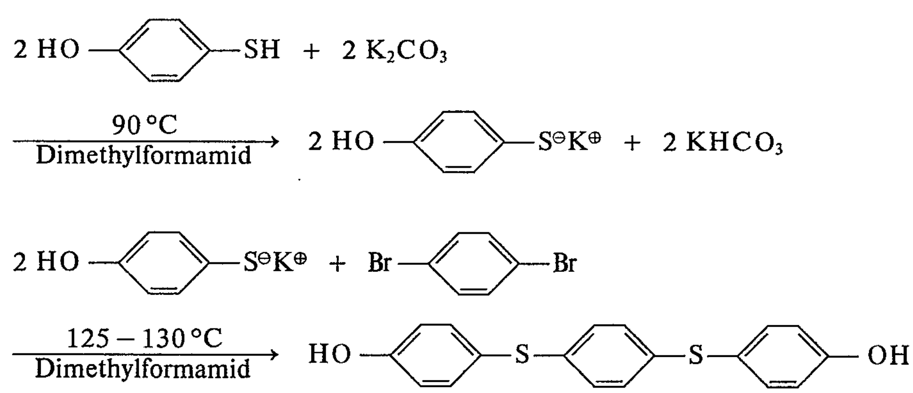


Wie das vorstehende Reaktionsschema zeigt, wird 4-Mercaptophenol mit p-Dibrombenzol in Gegenwart von Kaliumcarbonat und Dimethylformamid umgesetzt, wobei Bis-(4-hydroxyphenylthio)-benzol gemäß der Erfindung gebildet wird. Das erhaltene Monomere wird als Fällung isoliert und dann abgetrennt und getrocknet.

Wenn die Reaktion mit 0,1 Mol p-Dibrombenzol durchgeführt wird, wird nur das gewünschte Monomere in einer Ausbeute von 87,8% gebildet. Die Durchführung der Reaktion im größeren Maßstab auf der Basis von 3,0 Mol ergab ein Gemisch des gewünschten Monomeren und des erwarteten Nebenprodukts, des 4'-Brom-4-hydroxydiphenylsulfids, im Gewichtsverhältnis von 55 : 45.

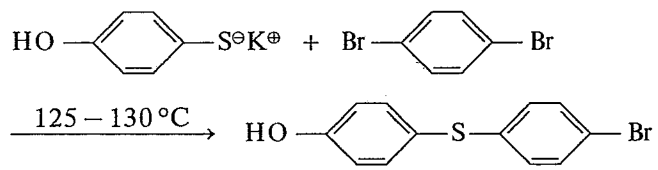


Die Abtrennung des Bis-(4-hydroxyphenylthio)-benzols vom 4'-Brom-4-hydroxydiphenylsulfid erfolgt durch Ausnutzung des Unterschiedes in der Löslichkeit. Das in Cyclohexan lösliche 4')-Brom-4-hydroxydiphenylsulfid wird durch Soxhlet-Extraktion des Reaktionsgemisches entfernt. Die Soxhlet-Extraktion ist ein bekanntes kontinuierliches Extraktionsverfahren, das in der organischen Chemie zur Trennung von Gemischen, die eine in einem Lösungsmittel lösliche Komponente enthalten, angewendet wird.

Das Bis-(4-hydroxyphenylthio)-benzol gemäß der Erfindung ist wertvoll als Monomer oder eines der Comonomeren für die Synthese von Polycarbonaten.

Diese Polycarbonate können unter Verwendung der neuen Monomeren gemäß der Erfindung nach bekannten Verfahren, wie sie beispielsweise in den US-PS 2 964 794, 2 970 131, 2 991 237, 2 999 835, 2 999 846, 3 028 365, 3 153 008, 3 187 065, 3 215 668 und 3 248 414 und in der Monographie von H. Schnell »Chemistry and Physics of Polycarbonates«, Interscience Publishers, New York 1964, beschrieben werden, hergestellt werden.

Beispiel 1

Synthese von Bis-(4-hydroxyphenylthio)-benzol

Zu einer Aufschlämmung von 910 g (6,59 Mol, 10%iger Überschuß) wasserfreiem Kalimcarbonat und 7 l destilliertem Dimethylformamid in einem 12-l-Rührwerks-Harzreaktor wurden 756 g (6,0 Mol) 4-Mercaptophenol gegeben. Die Reaktion wurde unter einer trockenen Stickstoffatmosphäre durchgeführt. Das Gemisch wurde langsam auf 60° C erhitzt. Zu diesem Zeitpunkt wurde Gelbfärbung des Reaktionsgemisches festgestellt. Dies ist auf die Bildung des Kaliumsalzes von 4-Mercaptophenol zurückzuführen. Das Reaktionsgemisch wurde 3 Stunden bei 90° C gehalten. Durch einen Zugabetrichter wurde ein Lösung von 708 g p-Dibrombenzol (3 Mol) in 1,5 l destilliertem Dimethylformamid dem Reaktionsgemisch in 30 Minuten zugesetzt. Die Reaktionstemperatur wurde auf 130° C erhöht und 20 Stunden bei diesem Wert gehalten. Das Reaktionsgemisch wurde zur Entfernung von Kaliumcarbonat und nicht umgesetztem Kaliumcarbonat durch einen Büchnertrichter filtriert. Der Rückstand im Büchnertrichter wurde mit 500 ml heißem Dimethylformamid gewaschen. Die vereinigten Filtrate wurden in drei gleiche Teile geteilt. Jeder Teil wurde in einen mit Rührer versehenen 19-l-Kunststoffkübel gegossen, der 11,36 l entsalztes Wasser und 175 ml konzentrierte Salzsäure enthielt. Nach Einstellung des pH-Werts auf 6 wurde der Inhalt des Kübels gekühlt und dann das Wasser aus dem Kübel dekantiert. Das hierbei erhaltene rohe Produkt, ein viskoses Material, wurde zweimal mit je 3,785 l Wasser gewaschen. Das hierbei erhaltene pulverförmige Material wurde abfiltriert und mit entsalztem Wasser säurefrei gewaschen. Das rohe Produkt wurde unter vermindertem Druck über Nacht bei 90° C getrocknet. (Schmelzpunkt 125—130° C).

Das aus der Reaktion (größerer Maßstab, Basis 3 Mol) erhaltene rohe Produkt enthielt Bis-(4-hydroxyphenylthio)-benzol und 4'-Brom-4-hydroxydiphenylsulfid im Gewichtsverhältnis von 55 : 45.

Zur Entfernung des 4'-Brom-4-hydroxydiphenylsulfids wurde das rohe Reaktionsprodukt mit heißem Cyclohexan 2 Stunden aufgeschlämmt und das erhaltene Gemisch filtriert. Drei gleiche Extraktionen wären erforderlich, um das 4'-Brom-4-hydroxydiphenylsulfid vom Bis-(4-hydroxyphenylthio)-benzol abzutrennen. Nach Entfernung des Cyclohexans vom Extrakt wurden 390 g 4'-Brom-4-hydroxydiphenylsulfid in weißen Flocken (Schmelzpunkt 78—79° C) erhalten. Der unlösliche Teil von 398 g erwies sich als 99%iges Bis-(4-hydroxyphenylthio)-benzol (Schmelzpunkt 158—160° C). Das Infrarotspektrum (IR) und das kernmagnetische Resonanzspektrum (NMR) stimmten mit den zugeordneten Strukturen überein. Die nachstehenden Werte der Elementaranalyse sind für die beiden Produkte ebenfalls im Einklang damit.

Bis-(4-hydroxyphenylthio)-benzol

| | | | | |
|---|---|---|---|---|
| Bruttoformel: | $C_{18}H_{14}S_2O_2$ (Molekulargewicht 326,292) | | | |
| Berechnet: | 66,26% C, | 4,32% H, | 19,6% S | |
| Gefunden: | 64,32% C, | 3,98% H, | 18,84% S | |

4'-Brom-4-hydroxydiphenylsulfid

| | | | | |
|---|---|---|---|---|
| Bruttoformel: | $C_{12}H_9BrSO$ (Molekulargewicht 281,192) | | | |
| Berechnet: | 51,25% C, | 3,23% H, | 11,38% S, | 28,45% Br |
| Gefunden: | 51,36% C, | 3,22% H, | 11,32% S, | 28,43% Br |

Beispiel 2

Herstellung eines Copolycarbonats aus Bisphenol A und Bis-(4-hydroxyphenylthio)-benzol

Ein Copolycarbonatharz wurde durch Umsetzung eines Gemisches der Dinatriumsalze von 2,2-Bis-(4-hydroxyphenyl)-propan (Bisphenol A) und Bis-(4-hydroxyphenylthio)-benzol mit Phosgen gemäß der Grenzflächen-Polykondensationssynthese hergestellt. 5 Gew.-% Bis-(4-hydroxyphenylthio)-benzol und 95 Gew.-% Bisphenol A, bezogen auf das Gewicht der Diphenole, wurden verwendet. Die für dieses Copolycarbonat gemessenen Eigenschaften sind in Tabelle 1 genannt.

Tabelle 1

| | |
|---|---|
| Izod-Kerbschlagzähigkeit[1] | |
| Dicke 3,18 mm | 817 J/M |
| Dicke 6,35 mm | 145 J/M |
| Kritische Dicke | 4,95 mm |
| Schmelzindex[2] | 6,70 g/10 Min. |
| Formbeständigkeit in der Wärme[3] | |
| Unter Belastung von 1,82 MPa | 120,8° C |
| Festigkeitseigenschaften[4] | |
| Zugfestigkeit an der Streckgrenze | 61 MPa |
| Reißfestigkeit (tensile strength, ultimate | 61 MPa |
| Zugfestigkeit beim Bruch (tensile strength, failure) | 49 MPa |
| Dehnung an der Streckgrenze | 8% |
| Bruchdehnung | 103% |
| Biegeeigenschaften[5] | |
| Biegefestigkeit | 86 MPa |
| Biegefestigkeit beim Bruch (Ultimate) | 86 MPa |
| Biegemodul | 2,3 GPa |
| Entflammbarkeitseigenschaften | |
| UL-94[6], Dicke 3,2 mm | 94 V-2 |
| Sauerstoffindex[7] | 23,9% |
| Optische Eigenschaften[8] | |
| Helligkeit | 83,26% |
| Vergilbungsindex beim Formen bei 288° C | 11,8% |
| Trübung beim Formen bei 288° C | 2,2% |
| Vergilbungsindex beim Formen bei 343° C | 17,4% |
| Helligkeit beim Formen bei 343° C | 80,5% |
| Schmelzviskosität, 300° C | |
| 7,2 Sek.$^{-1}$ | 620 Pa |
| 14,4 Sek.$^{-1}$ | 600 Pa |
| 36,0 Sek.$^{-1}$ | 540 Pa |
| 72,0 Sek.$^{-1}$ | 510 Pa |
| 144 Sek.$^{-1}$ | 510 Pa |
| 360 Sek.$^{-1}$ | 440 Pa |
| 720 Sek.$^{-1}$ | 360 Pa |
| 1440 Sek.$^{-1}$ | 290 Pa |
| Stabilität der Schmelze, 300° C | |
| 5 Min. | 510 Pa |
| 35 Min. | 370 Pa |
| 65 Min. | 350 Pa |
| (5—65) | 160 Pa |
| Relative Viskosität zu Beginn | 1,292 |
| Relative Viskosität des Spinnfadens nach 65 Min. (»strand RV«) | 1,279 |

[1] ASTM D-256
[2] ASTM D-2138 bei 300° C und 1200 g Belastung
[3] ASTM D-648/72
[4] ASTM D-638
[5] ASTM D-790
[6] Underwriters Laboratories, Inc. 94: Norm für die Prüfung von Kunststoffmaterialien für Teile in Vorrichtungen und Geräten auf Entflammbarkeit.
[7] ASTM D-2863
[8] ASTM D-1003

Die technische Verwendung der aus dem erfindungsgemäßen Bis-(4-hydroxyphenylthio)-benzol erhältlichen Polycarbonate kann überall dort erfolgen, wo die entsprechenden Polymeren auf Basis der bekannten Diarylsulfide bislang technische Verwendung finden konnten.

**Patentansprüche**

1. Bis-(4-hydroxyphenylthio)-benzol mit Strukturformel

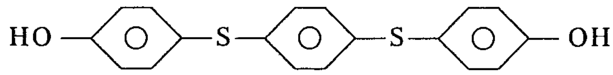

2. Verfahren zur Herstellung des Bis-(4-hydroxyphenylthio)-benzol des Anspruchs 1, dadurch gekennzeichnet, daß man 4-Mercaptophenol mit p-Dibrombenzol im Molverhältnis 2 : 1 in Gegenwart von Kaliumcarbonat und Dimethylformamid bei Temperaturen zwischen 90° C und 130° C umsetzt.

3. Verwendung des Bis-(4-hydroxyphenylthio)-benzols des Anspruchs 1 zur Herstellung von Polycarbonaten.

**Claims**

1. Bis-(4-hydroxyphenyl thio) benzene of the structural formula

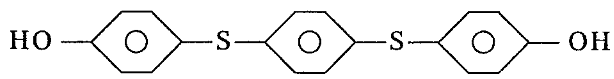

2. Process for the preparation of the bis-(4-hydroxyphenyl thio) benzene of Claim 1, characterised in that 4-mercaptophenol is reacted with p-dibromobenzene in a molar ratio of 2 : 1 in the presence of potassium carbonate and dimethyl formamide at temperatures between 90° C and 130° C.

3. Use of the bis-(4-hydroxyphenyl thio) benzene of Claim 1 in the preparation of polycarbonates.

**Revendications**

1. Le bis-(4-hydroxyphénylthio)-benzène de formule de structure

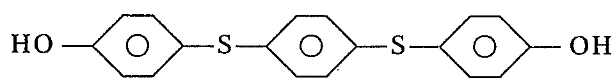

2. Procédé de préparation du bis-(4-hydroxyphénylthio)-benzène selon la revendication 1, caractérisé en ce que l'on fait réagir le 4-mercaptophénol avec le p-dibromobenzène dans un rapport molaire de 2 : 1 en présence de carbonate de potassium et de diméthylformamide à des températures de 90 à 130° C.

3. Utilisation du bis-(4-hydroxyphénylthio)-benzène selon la revendication 1 pour la préparation de polycarbonates.